# EUROPEAN PATENT APPLICATION

(11) **EP 1 649 873 A2**
(43) Date of publication of application: **26.04.2006**
(21) Application number: 05256587.6
(22) Date of filing: 24.10.2005
(51) Int. Cl.: A61L 15/60

(54) **Absorbent materials and articles**

(30) Priority: 22.10.2004 GB 0423485
(71) Applicant: First Water Limited, Marlborough, Wiltshire SN8 2RB (GB)
(72) Inventor: Munro, Hugh SEmple, Weston Sub Edge,Chipping Camden GL55 6QT (GB); Andrews, Philip, c/o -First Water Limited, Marlborough, Wiltshire SN8 2RB (GB); Sainz Garcia, Susana, Wantage, Oxforshire OX12 9XW (GB)
(74) Representative: Brown, David Leslie

(57) **Abstract**

An absorbent material comprises a flexible, skin-conformable, moisture-absorbent sheet member, optionally a net member in sheet form overlying and associated with the absorbent sheet member on at least one face thereof, and a hydrogel disposed on at least one of the net member, when present, and the absorbent sheet member in an amount of less than about 500 g of hydrogel per square metre per face, wherein the aqueous saline absorbency rate of the absorbent material through the face on which the hydrogel is disposed is less than about 300 seconds. Absorbent articles comprising the absorbent material and methods of manufacturing the material or articles are also described.

## Description

### Field of the Invention

The present invention relates to absorbent materials and articles.

More particularly, the invention relates to absorbent materials such as those suitable for use in wound dressings, and to absorbent articles such as wound dressings incorporating these materials.

The absorbent materials and articles most preferably exhibit rapid fluid uptake and moist wound healing properties.

The expression "wound" and like expressions, used herein, are intended to cover primarily - but not exclusively - skin lesions in human and other mammalian skin, for example cuts, grazes, abrasions, tears, bums, scalds, ulcers, spots, blisters. The wound can, for example, be dermal, epidermal, or a combination of both.

The term "skin" is to be understood generally, and includes for example the epidermis and dermis of human and other mammalian skin, as well as both mucosal and non-mucosal membranes.

### Background of the Invention

One traditional way of dealing with exudate from a wound has been by absorbing it using a wound dressing containing some type of absorbent material. Examples include dressings such as those shown in U.S. Patents Nos. 2893388, 3018881 and 3073304. All of these dressings contain a padded absorbent material attached to an adhesive tape backing. The padded absorbent material is applied to the wound to absorb the wound exudate.

A difficulty with this type of dressing is that as the wound heals, the scab typically forms in and as part of the pad. Thus, when the dressing is removed, the scab is removed.

U.S. Patents Nos. 2923298, 3285245 and 3870041 illustrate examples of products which have addressed this problem by providing a porous film between the absorbent material and the wound to reduce the likelihood that a scab formed will become attached to the absorbent material. U.S. Patent No. 3888247 discloses placing a microporous material over the wound and then applying a perforated urethane film containing a wound dressing made in accordance with U.S. Patent No. 3285245 over the microporous tape applied to the wound.

U.S. Patent No. 1967923 contains a cellulose sheet membrane or film which protects the dressing and allows air to circulate over the wound. Other wound dressings comprising films are disclosed in U.S. Patents Nos. 3645835, 4499896, 4598004, and 5849325.

A difficulty with dressings which comprise a thin film which is applied to the wound is "pooling" of exudate under the film if the wound is producing a large amount of exudate. This can result in loosening or removal of the wound dressing. An attempted solution to this problem is disclosed in U.S. Patent No. 1956695, which discloses a round plaster which contains a rubber film which expands to allow pus to collect under it. This plaster allows the exudate to remain against the wound. Another attempted solution is provided in U.S. Patent No. 3521631, which discloses an impervious sheet which is placed over a wound with an absorbent material extending over the impervious sheet and around its edges to allow wound exudate to pass into the absorbent material at the edges of the impervious sheet. This entire structure is covered with a backing sheet which is impervious and occlusive.

An alleged improvement of the device disclosed in U.S. Patent No. 3521631 is that disclosed in U.S. Patent No. 4181127. According to that disclosure, an imperforate film of polyurethane, which contacts the wound, has an absorbent material over it that overlaps the film edges so that the exudate passes into the absorbent material at the edges of the film. Adhesive tape can be applied over the top of the combination.

It is known that wound healing is assisted by a moist environment at the wound site. Moist wound healing is associated with a moisture-controlled environment that is believed to promote faster wound healing, reduced scarring and reduced pain. Wound dressings are known, for example, in which a hydrogel pad is incorporated into the dressing to provide a water reservoir in contact or communication with the wound site. See, for example, US Patents Nos. 5423737, 5480377, 5503847, 5571080 and 5693634, EP-A-0674498 and WO-A-97/24149,

However, hydrogels are relatively specialised and expensive materials, so that such wound care systems have traditionally not been used in sticking-plaster ("plaster") dressings used in the first aid treatment of cuts and abrasions, which are relatively cheap commodity consumer products. Even when hydrogels are used in specialised hospital products, the balance between maintenance of the hydrous environment on the skin surface and removal of infected, infectable or unpleasant exudate or sweat is difficult to achieve.

The prior art documents referred to above, and the documents cited as prior art in them, are incorporated herein by reference.

It is an object of the present invention to provide improved or at least alternative wound and skin dressings and other absorbent materials and articles.

### Brief Description of the Invention

According to a first aspect of the present invention, there is provided an absorbent material comprising a flexible, skin-conformable, moisture-absorbent fibrous sheet member which comprises a hydrogel disposed on at least one face of the sheet in an amount of less than about 500 g of hydrogel per square metre per face, wherein the aqueous saline absorbency rate of the absorbent material through the face on which the hydrogel is disposed is less than about 300 seconds, more preferably less than about 200 seconds.

The "aqueous saline absorbency" test referred to here involves placing a 1 ml drop of 0.9% w/w aqueous sodium chloride solution onto the material to be tested and measuring the time that elapses before the drop is fully absorbed. The test is performed under standard ambient conditions, for example room temperature and pressure (e.g. about 20 to 25°C; 1 atmosphere).

According to a second aspect of the present invention, there is provided an absorbent article, for example a wound dressing, comprising the absorbent material according to the first aspect of the invention.

The absorbent material may suitably further comprise a net member in sheet form overlying and associated with the absorbent sheet member on at least one face thereof. At least some of the hydrogel is disposed on the net member. The net member includes through-perforations to form a mesh. The net member is arranged to transport moisture through the perforations, whereas the absorbent sheet material is arranged to absorb moisture into its bulk without through-passage.

The use of a net member overlying and associated with an absorbent sheet member is, however, not limited to the case of a fibrous absorbent sheet member, and non-fibrous absorbent sheets, for example foam (e.g. open or partially open cell foam) sheets, can be used.

According to a third aspect of the present invention, therefore, there is provided an absorbent material comprising a flexible, skin-conformable, moisture-absorbent sheet member, a net member in sheet form overlying and associated with the absorbent sheet member on at least one face thereof, and a hydrogel disposed on at least one of the net member and the absorbent sheet member in an amount of less than about 500 g of hydrogel per square metre per face, wherein the aqueous saline absorbency rate of the absorbent material through the net member is less than about 300 seconds, more preferably less than about 200 seconds.

According to a fourth aspect of the present invention, there is provided an absorbent article, for example a wound dressing, comprising the absorbent material according to the third aspect of the invention.

According to a fifth aspect of the present invention, there is provided a sheet-form net member for use in manufacturing an absorbent material or article according to the invention which includes such a net member, wherein the net member includes substantially open through-perforations and comprises hydrogel material present in an amount of less than about 500 g of hydrogel per square metre of net.

According to a sixth aspect of the present invention, there is provided a method of manufacturing an absorbent material or article according to the foregoing aspects of the invention, which comprises applying an appropriate amount of the hydrogel to at least one face of an appropriate absorbent sheet material or net member or both. The said application of hydrogel may be carried out before, during or after any necessary manufacturing step of associating absorbent and net members together.

The said application of hydrogel to the absorbent sheet material or net member or both may suitably be by coating, impregnating, or a combination thereof. In this process, a precursor liquid (pregel) is preferably applied to the absorbent sheet material or net member or both, allowed to accumulate on the absorbent sheet material or net member or both, and/or be absorbed into it/them, and then the precursor liquid is treated so that the solid hydrogel is formed and/or deposited from the precursor liquid, as will be discussed in more detail below. The application process is such that the hydrogel does not substantially reduce the water uptake rate, as mentioned above. Generally, this will require either that the hydrogel does not occlude at least a substantial proportion of the surface moisture entry pores or other openings of the absorbent sheet member and/or the through-perforations of the net member, or that - if occlusion of pores or perforations does take place - sufficient regions of the occlusive hydrogel are thin enough that moisture can transport rapidly through them and thence into the absorbent sheet member. Furthermore, the maintenance of absorbency may require that the depth of impregnation of the precursor liquid into the structure of the absorbent sheet member is controlled, and generally it may be kept as low as possible.

In another embodiment, for example, hydrogel may be applied in an appropriate amount to a suitable sheet-form net structure in order to prepare a hydrogel-containing net member for use in the invention, and at least one said net member may subsequently be associated with a flexible, skin-conformable, moisture-absorbent sheet member, whereby the net member is disposed on at least one side of the absorbent sheet member.

The absorbent materials and articles of the present invention preferably have a rapid fluid uptake (typically measured as aqueous saline absorbency, i.e. the rate of uptake of a 1ml drop of 0.9% saline solution) of less than about 200 seconds. For example, the absorbency rate is suitably less than about 180 seconds, for example less than about 150 seconds, for example less than about 120 seconds, for example less than about 100 seconds, for example less than about 85 seconds, preferably less than about 60 seconds, for example less than about 45 seconds, for example less than about 30 seconds, for example less than about 15 seconds, and most preferably less than about 10 seconds.

The absorbent material and articles have a generally low amount of hydrogel material in them. This makes the costs manageable for mass-market "sticking-plaster" and other consumer absorbent articles, and also enables the sheet materials to be easily rolled on themselves for storage and transportation, particularly in the manufacturing stages. Indeed, the amount of hydrogel present may be so low that in many cases a protective release layer may not be required in order to protect the hydrogel from contamination, from sticking to other things or to other parts of the same roll of material. This ease of handling provides a further significant advantage of the invention.

Furthermore, we have surprisingly found that there is a beneficial interaction between the hydrogel and the absorbent sheet member to which it is applied, in that a certain amount of water is apparently abstracted from the hydrogel by the absorbent sheet, causing a lowering of the observed water activity of the hydrogel. This provides evidence of an active water transport mechanism within the absorbent material of the present invention.

The absorbent material and articles of the present invention have a number of uses and applications, as described in more detail below. In particular, the invention enables cost-effective mass-market wound dressing products with moist wound healing properties.

### Detailed Description of the Invention

### Fibrous Absorbent Sheet Members

Where the absorbent sheet member is fibrous, this is preferably a coherent structure comprised of fibres, the structure being capable of being swollen by aqueous fluid, that are held together (e.g. by interweaving, entangling, adhesion, compaction, partial melting together or a combination thereof) to maintain overall coherency of the structure. The expression "fibres" includes all elongate forms such as strips, strands and threads. The fibres may be of unitary construction (e.g. by extrusion) or may be composed of a plurality of smaller filaments, which themselves may be secured together in the fibre by any appropriate means, e.g. by intertwining, entangling, spinning, adhesion, partial melting together or a combination thereof. Examples of such structures are knitted, woven and non-woven materials such as felts, mats and the like.

The fibres and/or filaments can be of constant transverse cross-sectional configuration along their length or a portion thereof, or the transverse cross-sectional configuration of the fibres and/or filaments can vary along their length randomly or regularly. The transverse cross-sectional configuration at any particular point along the length of a particular fibre or filament can be any appropriate shape, including square, rectangular, triangular, polygonal, circular, oval, ellipsoidal, irregular, any of the above with indentations, any of the above with projections, or an approximation to any of the above.

The fibres of the fibrous material may themselves be absorbent, so that swelling of the fibrous material would then include swelling of individual fibres through uptake of water into the fibres.

Where the fibrous absorbent member comprises a non-woven material, this material may be selected from, for example, needlepunched, spunlaced or hydroentangled non-wovens, airlaid, spunbonded and spunmelt non-woven composites, and melt blown, carded (long and short staple), wet laid, thermal bonded, chemical bonded, resin bonded, thru-air bonded and stitch-bonded porous film non-wovens and any combination thereof.

The fibres are suitably formed from materials selected from natural, synthetic or part-natural, part-synthetic materials or combinations thereof, for example cotton, glass, polyamides (e.g. Nylon™), polyolefins (e.g. polyethylene and polypropylene), polyester, cellulose, carboxymethyl cellulose, alginates (e.g. calcium alginate) and polyacrylates (e.g. sodium polyacrylate) used singly or blended in any combination thereof. Such materials are particularly suitable for non-woven fibrous sheets. Such a non-woven material may comprise both fibre and powder (for example sodium alginate and carboxymethyl cellulose). Suppliers of suitable non-wovens include Lantor UK, BFF and Technical Absorbents Limited, Acordis Speciality Fibres.

The fibrous absorbent member may, for example, be essentially free of hydrogel material prior to application of the hydrogel to the surface of the absorbent member in accordance with the invention.

The fibrous absorbent sheet member (e.g. non-woven) preferably has one or more of the following characteristics:-
weight between about 10 and about 500 grams per square metre (gsm), preferably between about 20 and about 400gsm and even more preferably between about 30 and about 350gsm;
thickness (major face to major face) less than about 5mm, e.g. between about 0.05 and about 5mm, preferably less than about 2mm and even more preferably less than about 1.75mm;
aqueous saline absorbency rate less than about 60s, preferably less than about 40, preferably less than about 20s and more preferably less than about 10s;
water uptake, as determined by the %weight increase in a 1 cm² sample on immersion in 100ml of deionised water for 10 minutes is preferably greater than about 50%, preferably greater than about 100% preferably greater than about 200 %. In cases where heavily exuding wounds are to treated then the water uptake should be preferably greater than about 500%, for example greater than about 1000%.

These preferred one or more characteristics of the absorbent sheet member are also preferred one or more characteristics of the absorbent sheet mamber after application of the hydrogel thereto.

The fibrous sheet material (e.g. non-woven) may be embossed or perforated.

### Other Absorbent Sheet Members

Where the absorbent sheet member is non-fibrous, it may preferably comprise an absorbent foam, membrane or film. A foam may be closed cell, open cell or partially-closed, partially-open cell, with open cell being preferred because of the absorbency.

The sheet member is suitably formed from absorbent materials selected from natural, synthetic or part-natural, part-synthetic materials or combinations thereof, for example polyamides (e.g. Nylon™), polyurethane, polyolefins (e.g. polyethylene and polypropylene), polyester, cellulose, carboxymethyl cellulose, alginates (e.g. calcium alginate) and polyacrylates (e.g. sodium polyacrylate) used singly or blended in any combination thereof, and optionally foamed.

The absorbent sheet member may, for example, be essentially free of hydrogel material prior to application of the hydrogel to the surface of the absorbent member in accordance with the invention.

Alternatively, the absorbent sheet member may comprise a hydrogel foam or hydrogel-containing foam, provided that it has the required mechanical and water absorbency properties. Examples of such hydrogel foams are given in WO-A-03/077964 and the prior art documents acknowledged therein and in the International Search Report established thereon, as well as US Patent No. 6174929 and the prior art documents acknowledged therein, the contents of all of which are incorporated herein by reference.

The absorbent sheet member preferably has one or more of the same weight, thickness, aqueous saline absorbency rate and water uptake characteristics as the fibrous absorbent sheet materials mentioned above.

### Other Optional Sheet Components

The absorbent sheet member may if desired further comprise other optional sheet components such as, for example, a moisture permeable or impermeable backing layer possessing a front, skin-facing, surface and a back, garment-facing, surface. The backing layer may overlie the absorbent sheet member layer and the margins of the layers may be coterminous, one may extend beyond the margins of the other, or in certain regions of the assembly the margins of the layers may be coterminous and in other regions one layer may extend beyond the margins of the other. The backing layer may, for example, extend beyond the margins of the absorbent sheet layer. The backing layer preferably carries a skin-compatible adhesive on at least those portions of its front surface which extend beyond the margins of the absorbent sheet layer, whereby the assembly can be stuck to a wearer's skin in use.

The backing layer, when present, may for example be formed from a natural or synthetic polymer or any combination or blend thereof. Examples of such polymers include, without limitation, polyurethane films, natural fabrics, synthetic fabrics, polyolefins and polyesters.

The backing layer, when present, may, for example, be essentially free of hydrogel material.

The multi-layer structure may suitably comprise a pressure sensitive adhesive, for example an acrylic or hydrogel based adhesive, which may be coated on the side of the backing layer in contact with the absorbent material to act as a means of more securely attaching the backing layer to the absorbent material. As mentioned above, when the adhesive coated backing layer exceeds the area dimensions of the absorbent material, the adhesive may suitably function as a means for attachment of the assembled structure to the skin.

### Optional Perforations

The absorbent sheet member and any associated backing or other layers may be in the form of a continuous sheet or perforated. The perforations, when present, may be of any shape, for example - but not limited to, circular, square, rectangular, triangular, polygonal, circular, oval, ellipsoidal, irregular, any of the above with indentations, any of the above with projections, or an approximation to any of the above. The side walls of the perforations may be tapered in a straight way, tapered in a curved way, untapered, or any combination thereof at different points along their length. The perforations may include regions along their lengths which define enlarged cavities within the material structure. The perforations may be interconnected within the material structure, and such interconnections may comprise passages which may, for example, have tapering side walls which taper in a straight way, tapering side walls which taper in a curved way, untapered side walls, side walls which define enlarged cavities within the material structure, or any combination thereof at different points along their length.

The size and frequency of any perforations may be varied according to requirements, aesthetic and functional. The transverse cross-sectional area of each perforation as appearing at the surface of the absorbent sheet member will be significantly smaller than the cross-sectional area of the openings of any net member, so that the respective functions of the net and the absorbent member are not impeded.

The perforations, when present, may be provided in a regular array across the absorbent sheet member, or may be irregularly provided, or at least one region of perforations may be regular and at least one other region may be irregular. The perforations may define indicia, for example letters, numbers, shapes, logos

### Optional Net Member

The net member, when present, will contact the wound or skin of the wearer in use.

It is therefore generally important that the net member, when present, does not substantially hinder the absorbency of the absorbent sheet member.

The net member should preferably be of a substantially more open foraminous structure than the absorbent sheet member. Preferably, the net member will have perforation sizes of between about 0.01 and about 25 mm², preferably between about 0.05 and about 10mm² and more preferably between about 0.1 and about 1mm².

The size and frequency of any perforations may be varied according to requirements, aesthetic and functional. The transverse cross-sectional area of each perforation as appearing at the surface of the absorbent sheet member may suitably be less than about 9 cm², for example less than about 7 cm², for example less than about 4 cm², for example less than about 1 cm².

The net member will suitably be formed in conventional manner from polymers such as those selected from polyolefins (e.g. polyethylene and polypropylene), polyamides (e.g. Nylon™), ethylene/vinyl acetate (EVA) copolymers and combinations and blends thereof. The net material is preferably of low adherence to a wound. It may preferably be a polyolefin or polyolefin-containing blend, for example high density polyethylene (HDPE) or a blend such as, for example, HDPE/Surlyn® or HDPE/EVA.

The net member may, for example, be essentially free of hydrogel material prior to application of any hydrogel to the surface of the net member in accordance with the invention.

A number of absorbent sheet products are commercially available, which comprise a flexible, skin-conformable non-woven absorbent sheet member associated with an overlying net member. Such products are particularly suitable for use in the present invention. Examples of such products include those available from Lantor under the Lantor reference number 50.03.18, 47.02.09, 71.01.06, 46.09.03 and 50.03.18 and those available from Freudenberg under the Freudenberg reference M1561.

The net member may be formed in conventional manner from fibres that are held together (e.g. by interweaving, entangling, adhesion, compaction, partial melting together or a combination thereof) to maintain overall coherency of the net. The expression "fibres" includes all elongate forms such as strips, strands and threads. The fibres may be of unitary construction (e.g. by extrusion) or may be composed of a plurality of smaller filaments, which themselves may be secured together in the fibre by any appropriate means, e.g. by intertwining, entangling, spinning, adhesion, partial melting together or a combination thereof.

The fibres and/or filaments of the net may have any of the characteristics mentioned above in the discussion of the fibres of the fibrous absorbent sheet member.

### The Application of the Hydrogel

The amount of hydrogel present on the absorbent and/or the net member (when present) is preferably based on the ready-to-use weight of hydrogel, i.e. in its hydrated form. The hydrogel is present on at least the skin-directed face of the absorbent material or articles according to the present invention.

It is an important feature of the invention that the hydrogel is used in rather low amounts. More preferably, the hydrogel will be present in an amount of less than about 400 g of hydrogel per square metre per face, for example less than about 300 g per square metre per face and most preferably between about 50 and about 250 g per square metre per face.

The hydrogel is preferably applied by coating, impregnation or a combination thereof. One aspect in the choice of application method will be the absorbency of the substrate onto which the hydrogel is to be applied. If that substrate is a relatively impermeable net member, coating may be preferred. If, however, that substrate is or includes an absorbent sheet member, impregnation of the hydrogel may be preferred.

The coating or impregnation typically comprises contacting the substrate with a liquid precursor (pregel) formulation which is curable or evaporatable to form the hydrogel, allowing a desired amount of the precursor formulation to accumulate on the substrate surface or to be absorbed to a desired depth into the substrate bulk, and then curing or evaporating the precursor formulation in known manner to form the hydrogel as a solid product or residue.

Further details of the formation of the hydrogel and the precursor fluid are given below in the discussion of the hydrogel material and the method of applying it to the absorbent and/or net structure.

Absorbent sheet members and/or net members may have hydrogel applied to them on one or both major faces.

The hydrogel as applied to the absorbent and/or net structure is preferably is largely or completely non-continuous, by which is meant that areas of untreated absorbent sheet member or net material (particularly the absorbent sheet member as the openings in the net are less likely to occlude) are left exposed or sufficiently thinly covered by hydrogel for rapid fluid flow connection with the wearer's skin or the wound site, so that fluid (e.g. wound exudate) contacting the hydrogel treated surface is rapidly absorbed, e.g. at a rate not substantially different from the absorbency rate of the corresponding structure without the hydrogel.

The extent of non-continuity of the hydrogel on the absorbent sheet member and/or net member is preferably such that 1 ml of saline is absorbed into the absorbent sheet member from the hydrogel-treated side in less than about 5 minutes, for example less than about 3 minutes, preferably less than about one minute.

If desired, the hydrogel may be applied to a net member as such or to the net side of a multi-layer composite of the absorbent sheet member and the net member. In either case the resultant hydrogel-containing product may subsequently be incorporated into an absorbent material or article according to the invention.

However, it is in some cases preferable for a controlled partial occlusion of the perforations of a net member to be arranged, within the general limitation set out above. For example, if a portion of the liquid precursor for the hydrogel is allowed or made to pass through some or all of the holes of the net, it can then to some extent contact, and/or be absorbed into, any underlying absorbent sheet member present during the hydrogel application process, or - if the hydrogel is being applied to the net member as such - drips or projections of hydrogel material can be formed on the side of the net which will later be brought into contact with the absorbent sheet material. In either case, a "hydrogel bridge" can thus be established between the net member and the absorbent sheet member - and thereby a continuous fluid-transport bridge between the wearer's skin or wound site and the absorbent sheet member. Such a fluid transport bridge into the absorbent sheet material is believed to offer potentially significant benefits in establishing and maintaining an effective moisture cycle within the dressing in use, whereby a moist environment is maintained in the vicinity of the wound through balancing and equilibration of such factors as any one or more of: exudation from the wound, sweating by the skin, absorption of exudate and/or sweat by the absorbent sheet material, absorption of external and/or atmospheric moisture by the absorbent sheet material, absorption of exudate and/or sweat by the hydrogel, absorption of external and/or atmospheric moisture by the hydrogel, evaporation of exudate and/or sweat from the absorbent sheet material, evaporation of external and/or atmospheric moisture from the absorbent sheet material, evaporation of exudate and/or sweat from the hydrogel, and evaporation of external and/or atmospheric moisture from the hydrogel.

### The Nature of the Hydrogel

The expression "hydrogel" and like expressions, used herein, are not to be considered as limited to gels which contain water, but extend generally to all hydrophilic gels, including those containing organic non-polymeric components in the absence of water. The gel forming agent may, for example, be selected from natural hydrophilic polymers, synthetic hydrophilic polymers, hydrocolloids, gelling hydrophilic biopolymers and all combinations thereof.

Hydrogels are, generally speaking, hydrophilic polymers characterized by their hydrophilicity (i.e capacity to absorb large amounts of fluid such as wound exudate) and insolubility in water: i.e. they are capable of swelling in water while generally preserving their shape.

The hydrophilicity is generally due to groups such as hydroxyl, carboxy, carboxamido, and esters, among others. On contact with water, the hydrogel assumes a swollen hydrated state that results from a balance between the dispersing forces acting on hydrated chains and cohesive forces that do not prevent the penetration of water into the polymer network. The cohesive forces are most often the result of crosslinking, but may result from electrostatic, hydrophobic or dipole-dipole interactions.

Useful classes of hydrogels in the present invention include those polymers and copolymers derived from acrylic and methacrylic acid ester, including hydroxyalkyl (meth)acrylates, 2-(N,N-dimethylamino)ethyl methacylate, ω-methacryloyloxyalkyl sulfonates (generally crosslinked with diacrylate or divinylbenzene), polymers and copolymers of substituted and unsubstituted acrylamides, polymers and copolymers of N-vinylpyrrolidinone, and polyelectrolyte complexes. Hydrogels are described in greater detail in Hydrogels, Kirk-Othmer Encyclopedia of Chemical Technology, 4^{th} Edition, vol. 7, pp. 783-807, John Wiley and Sons, New York, the contents of which are incorporated herein by reference.

The term "hydrogel" is used herein regardless of the state of hydration.

The hydrogel used in connection with the present invention will suitably comprise a substantially water-insoluble, slightly crosslinked, partially neutralized, gel-forming polymer material. Such polymer materials can be prepared from polymerisable, unsaturated, acid- and ester-containing monomers.

Thus, such monomers include the olefinically unsaturated acids, esters and anhydrides which contain at least one carbon to carbon olefinic double bond. More specifically, these monomers can be selected from olefinically unsaturated carboxylic acids, carboxylic esters, carboxylic acid anhydrides; olefinically unsaturated sulphonic acids; and mixtures thereof.

Olefinically unsaturated carboxylic acid, carboxylic acid ester and carboxylic acid anhydride monomers include the acrylic acids typified by acrylic acid itself, methacrylic acid, ethacrylic acid, α-chloroacrylic acid, α-cyano-acrylic acid, β-methyl-acrylic acid (crotonic acid), α-phenyl acrylic acid, β-acryloxy-propionic acid, sorbic acid, α-chloro-sorbic acid, angelic acid, cinnamic acid, *p*-chlorocinnamic acid, β-styryl-acrylic acid (1-carboxy-4-phenyl-1,3-butadiene), itaconic acid, citraconic acid, mesaconic acid, glutaconic acid, aconitic acid, maleic acid, fumaric acid, tricarboxy-ethylene and maleic acid anhydride and salts (e.g. alkali metal salts such as sodium, potassium and lithium salts) thereof. Olefinically unsaturated sulphonic acid monomers include aliphatic or aromatic vinyl sulphonic acids such as vinylsulphonic acid, allylsulphonic acid, vinyltoluenesulphonic acid and styrene sulphonic acid; acrylic and methacrylic sulphonic acid such as sulphoethyl acrylate, sulphoethyl methacrylate, sulphopropyl acrylate, sulphopropyl methacrylate, 2-hydroxy-3-acryloxy propyl sulphonic acid, 2-hydroxy-3-methacryloxy propyl sulphonic acid and 2-acrylamido-2-methyl propane sulphonic acid and salts (e.g. ammonium or alkali metal salts such as sodium, potassium and lithium salts) thereof.

Further examples of suitable monomers for use in the present invention include:
a polyalkylene glycol acrylate or a substituted derivative thereof; a polyalkylene glycol methacrylate or a substituted derivative thereof; acrylic acid (3-sulphopropyl) ester or a substituted derivative thereof or a salt thereof (e.g. an alkali metal salt such as sodium, potassium or lithium salt); diacetone acrylamide (N-1,1-dimethyl-3-oxobutyl-acrylamide); a vinyl lactam (e.g. N-vinyl pyrrolidone or a substituted derivative thereof); an optionally substituted N-alkylated acrylamide such as hydroxyethyl acrylamide; and an optionally substituted N,N-dialkylated acrylamide; and/or N-acryloyl morpholine or a substituted derivative thereof.

The above monomers and monomer types may optionally include substituent groups. Optional substituents of the monomers used to prepare the hydrogels used in the present invention may preferably to selected from substituents which are known in the art or are reasonably expected to provide polymerisable monomers which form hydrogel polymers having the properties necessary for the present invention. Suitable substituents include, for example, lower alkyl, hydroxy, halo and amino groups.

The hydrogel used in the present invention preferably comprises a plasticised three-dimensional matrix of cross-linked polymer molecules, and has sufficient structural integrity to be self-supporting even at very high levels of internal water content, with sufficient flexibility to conform to the surface contours of mammalian skin or other surface with which it is in contact.

The hydrogel generally comprises, in addition to the cross-linked polymeric network, an aqueous or non-aqueous plasticising medium including an organic plasticiser. This plasticising medium is preferably present in the same precursor solution as the monomer(s).

The precursor liquid can comprise a solution of the gel-forming polymer in a relatively volatile solvent, whereby the hydrogel is deposited as a residue on evaporation of the solvent, or - more preferably - the precursor liquid will comprise a solution of the monomer(s), cross-linking agent, plasticiser, and optionally water and other ingredients as desired, whereby the hydrogel is formed by a curing reaction performed on the precursor liquid after application to the substrate to which the hydrogel is to be applied.

In the following discussion, the second form of precursor solution and application protocol (*in situ* polymerisation of the hydrogel) will be discussed. The solvent deposition method carried out on a pre-formed gel-forming polymer is well known and the details of that procedure do not need to be reproduced here.

The polymerisation reaction is preferably a free-radical polymerisation with cross-linking, which may for example be induced by light, heat, radiation (e.g. ionising radiation), or redox catalysts, as is well known.

For example, the free radical polymerisation may be initiated in known manner by light (photoinitiation), particularly ultraviolet light (UV photoinitiation); heat (thermal initiation); electron beam (e-beam initiation); ionising radiation, particularly gamma radiation (gamma initiation); non-ionising radiation, particularly microwave radiation (microwave initiation); or any combination thereof. The precursor solution may include appropriate substances (initiators), at appropriate levels, e.g. up to about 5% by weight, more particularly between about 0.002% and about 2% by weight, which serve to assist the polymerisation and its initiation, in generally known manner.

Preferred photoinitiators include any of the following either alone or in combination:
Type I-α-hydroxy-ketones and benzilidimethyl-ketals e.g. Irgacure 651. These are believed on irradiation to form benzoyl radicals that initiate polymerisation. Photoinitiators of this type that are preferred are those that do not carry substituents in the *para* position of the aromatic ring.
A particularly preferred photoinitiator is 1-hydroxycyclohexyl phenyl ketone; for example, as marketed under the trade name Irgacure 184 by Ciba Speciality Chemicals. Also preferred are Daracur 1173 (2-hydroxy-2-propyl phenyl ketone) and mixtures of Irgacure 184 and Daracur 1173.
Photo-polymerisation is particularly suitable, and may be achieved using light, optionally together with other initiators, such as heat and/or ionizing radiation. Photoinitiation will usually be applied by subjecting the pre-gel reaction mixture containing an appropriate photoinitiation agent to ultraviolet (UV) light. The incident UV intensity, at a wavelength in the range from 240 to 420nm, is typically greater than about 10mW/cm². The processing will generally be carried out in a controlled manner involving a precise predetermined sequence of mixing and thermal treatment or history.
The UV irradiation time scale should ideally be less than 60 seconds, and preferably less than 10 seconds to form a gel with better than 95% conversion of the monomers. Those skilled in the art will appreciate that the extent of irradiation will be dependent on a number of factors, including the UV intensity, the type of UV source used, the photoinitiator quantum yield, the amount of monomer(s) present, the nature of the monomer(s) present and the presence of polymerisation inhibitor.
In one preferred embodiment, (on the one hand) the precursor solution in contact with the substrate to which it is to be applied and (on the other hand) the source of the polymerisation initiator (e.g. the radiation source) may move relative to one another for the polymerisation step. In this way, a relatively large amount of polymerisable material can be polymerised in one procedure, more than could be handled in a static system. This moving, or continuous, production system is preferred.
After completion of the polymerisation, the product is preferably sterilised in conventional manner. The sterile composite may be used immediately, e.g. to provide a skin-adhesive layer in an article, or a top release layer may be applied to the composite for storage and transportation of the composite.
If desired, certain ingredients of the hydrogel may be added after the polymerisation and optional cross-linking reaction. However, it is generally preferred that substantially all of the final ingredients of the hydrogel are present in the precursor solution, and that - apart from minor conventional conditioning or, in some cases, subsequent modifications caused by the sterilisation procedure - substantially no chemical modification of the hydrogel takes place after completion of the polymerisation reaction.

### Monomers

Particularly preferred monomers include: the sodium salt of 2-acrylamido-2-methylpropane sulphonic acid, commonly known as NaAMPS, which is available commercially at present from Lubrizol as either a 50% aqueous solution (reference code LZ2405) or a 58% aqueous solution (reference code LZ2405A); acrylic acid (3-sulphopropyl) ester potassium salt, commonly known as SPA or SPAK (SPA or SPAK is available commercially in the form of a pure solid from Raschig); N-acryloyl morpholine; and hydroxyethyl acrylamide.

### Cross-linking Agents

Conventional cross-linking agents are suitably used to provide the necessary mechanical stability and to control the adhesive properties of the hydrogel. The amount of cross-linking agent required will be readily apparent to those skilled in the art such as from about 0.01% to about 0.5%, particularly from about 0.05% to about 0.4%, most particularly from about 0.08% to about 0.3%, by weight of the total polymerisation reaction mixture. Typical cross-linkers include tripropylene glycol diacrylate, ethylene glycol dimethacrylate, triacrylate, polyethylene glycol diacrylate (polyethylene glycol (PEG) molecular weight between about 100 and about 4000, for example PEG400 or PEG600), and methylene bis acrylamide.

### Organic Plasticisers

The one or more organic plasticiser, when present, may suitably comprise any of the following either alone or in combination: at least one polyhydric alcohol (such as glycerol, polyethylene glycol, or sorbitol), at least one ester derived therefrom, at least one polymeric alcohol (such as polyethylene oxide) and/or at least one mono- or poly-alkylated derivative of a polyhydric or polymeric alcohol (such as alkylated polyethylene glycol). Glycerol is the preferred plasticiser. An alternative preferred plasticiser is the ester derived from boric acid and glycerol. When present, the organic plasticiser may comprise up to about 45% by weight of the hydrogel composition.

### Surfactants

Any compatible surfactant may optionally be used as an additional ingredient of the hydrogel composition. Surfactants can lower the surface tension of the mixture before polymerisation and thus aid processing. The surfactant or surfactants may be non-ionic, anionic, zwitterionic or cationic, alone or in any mixture or combination. The surfactant may itself be reactive, i.e. capable of participating in the hydrogel-forming reaction. The total amount of surfactant, if present, is suitably up to about 10% by weight of the hydrogel composition, preferably from about 0.05% to about 4% by weight.

In a preferred embodiment of the invention the surfactant comprises at least one propylene oxide/ethylene oxide block copolymer, for example such as that supplied by BASF Plc under the trade name Pluronic P65 or L64.

### Other additives

The hydrogel in the composite of the present invention may include one or more additional ingredients, which may be added to the pre-polymerisation mixture or the polymerised product, at the choice of the skilled worker. Such additional ingredients are selected from additives known in the art, including, for example, water, organic plasticisers, surfactants, polymeric material (hydrophobic or hydrophilic in nature, including proteins, enzymes, naturally occurring polymers and gums), synthetic polymers with and without pendant carboxylic acids, electrolytes, pH regulators, colorants, chloride sources, bioactive compounds and mixtures thereof. The polymers can be natural polymers (e.g. xanthan gum), synthetic polymers (e.g. polyoxypropylene-polyoxyethylene block copolymer or poly-(methyl vinyl ether *alt* maleic anhydride)), or any combination thereof. By "bioactive compounds" we mean any compound or mixture included within the hydrogel for some effect it has on living systems, whether the living system be bacteria or other microorganisms or higher animals such as the patient. Bioactive compounds that may be mentioned include, for example, pharmaceutically active compounds, antimicrobial agents, antiseptic agents, antibiotics and any combination thereof. Antimicrobial agents may, for example, include: sources of oxygen and/or iodine (e.g. hydrogen peroxide or a source thereof and/or an iodide salt such as potassium iodide) (see, for example Bioxzyme™ technology, for example in The Sunday Telegraph (UK) 26 January 2003 or the discussion of the Oxyzyme™ system at www.wounds-uk.com/posterabstracts2003.pdf); honey (e.g. active Manuka honey); antimicrobial metals, metal ions and salts, such as, for example, silver-containing antimicrobial agents (e.g. colloidal silver, silver oxide, silver nitrate, silver thiosulphate, silver sulphadiazine, or any combination thereof); or any combination thereof.

In the Bioxzyme system, a dressing comprises two hydrogels. One contains glucose based antibacterial compounds and the other contains enzymes that convert the glucose into hydrogen peroxide. When these are exposed to air and contacted together at a wound site, the enzyme-containing gel being adjacent the skin and the glucose-containing gel overlying the enzyme-containing gel, a low level steady flow of hydrogen peroxide is produced, which inhibits anaerobic bacteria. This antibacterial effect can be enhanced by the inclusion of a very low level of iodide (less than about 0.04%) in the hydrogel. The hydrogen peroxide and the iodide react to produce iodine, a potent antimicrobial agent.

Hydrogels incorporating antimicrobial agents may, for example, be active against such organisms as *Staphylococcus aureus* and *Pseudomonas aeruginosa*.

Agents for stimulating the healing of wounds and/or for restricting or preventing scarring may be incorporated into the hydrogel. Examples of such agents include growth factors e.g. from GroPep Ltd, Australia or Procyte, USA (see, e.g. WO-A-96/02270, the contents of which are incorporated herein by reference); cell nutrients (see, e.g., WO-A-93/04691, the contents of which are incorporated herein by reference); glucose (see, e.g., WO-A-93/10795, the contents of which are incorporated herein by reference); an anabolic hormone or hormone mixture such as insulin, triiodothyronine, thyroxine or any combination thereof (see, e.g., WO-A-93/04691, the contents of which are incorporated herein by reference); or any combination thereof.

Additional polymer(s), typically rheology modifying polymer(s), may be incorporated into the polymerisation reaction mixture at levels typically up to about 10% by weight of total polymerisation reaction mixture, e.g. from about 0.2% to about 10% by weight. Such polymer(s) may include polyacrylamide, poly-NaAMPS, polyethylene glycol (PEG), polyvinylpyrrolidone (PVP) or carboxymethyl cellulose.

The hydrogel used in the present invention preferably consists essentially of a cross-linked hydrophilic polymer of a hydrophilic monomer and optionally one or more comonomer, together with water and/or one or more organic plasticiser, and optionally together with one or more additives selected from surfactants, polymers, pH regulators, electrolytes, chloride sources, bioactive compounds and mixtures thereof, with less than about 10% by weight of other additives.

For further details of suitable hydrogel material for use in the present invention, and its preparation, please refer to the following publications: PCT Patent Applications Nos. WO-97/24149, WO-97/34947, WO-00/06214, WO-00/06215, WO-00/07638, WO-00/46319, WO-00/65143 and WO-01/96422, the disclosures of which are incorporated herein by reference.

The water activity, which is related to the osmolarity and the ionic strength of the precursor solution (as measured, for example, by a chilled mirror dewpoint meter, Aqualab T3) is preferably beween 0.05 and 0.99, more preferably between, 0.2 and 0.99, and even more preferably between 0.3 and 0.98. The higher the ionic strength, reflected in a lower water activity, the lesser the swelling of the fibre structure. The ionic strength of the precursor solution can therefore be used to optimise the hydrogel composite properties.

### Application of the Hydrogel to the Absorbent and/or Net Sheet

As briefly mentioned already, a precursor liquid for the hydrogel is contacted with the component to which the hydrogel is to be applied. This contacting may be achieved in any convenient manner. For example, an absorbent or net sheet may be dipped into a bath of solution or the solution may be dispensed onto the sheet from, for example, a slot die.

Alternatively, the precursor solution may be dispensed onto an impervious substrate such as, for example, a release layer coated with a non-stick material, and the component to which the hydrogel is to be applied is then placed on top of the solution. This method may be particularly suitable where the component to which the hydrogel is to be applied is absorbent, using the absorbency characteristics of the component to take up the precursor solution into its bulk.

Generally speaking, any conventional liquid coating or application technique can be used, including gravure, roll, reverse roll, reverse gravure, screen printing and slot die coating.

The precursor liquid preferably has a viscosity less than about 20,000 centipoise (cps), preferably less than about 10,000 cps, and more preferably less than about 1,000 cps and even more preferably less than about 100 cps. The amount of hydrogel liquid applied to achieve a suitably non-continuous coating is dependent on its viscosity and the nature of the absorbent layer but is preferably less than about 500 grams per square meter (gsm), preferably less than about 300, preferably less than about 200 and even more preferably less than about 150 gsm. These application weights correspond closely to the application weights of the cured hydrogel, as in the preferred *in situ* polymerisation process little weight is lost in the curing reaction.

The length of time between applying the precursor solution and curing (polymerising and optionally crosslinking) the composite may be varied to allow control over the extent of fibre swelling and resultant properties for example fluid uptake and strength of the swollen composite. Preferably, the length of time the precursor solution is in contact with the fibre before curing is between 0.5 and 45 seconds, more preferably between 1 and 20 seconds.

The nature and extent of impregnation of the fibrous material by the precursor solution can thus be varied extensively according to the desired characteristics of the final composite material. For example, there can be a gradient, which can be linear or non-linear or part-linear-part-non-linear, of the amount (e.g. by weight) of the precursor solution taken up per unit volume of fibrous material, according to the distance into the bulk of the fibrous material. That gradient will be such that, at any particular region or regions within the fibrous material, the amount of precursor solution per unit volume of fibrous material increases or decreases with distance into the bulk of the fibrous material. Alternatively, regions or the whole of the bulk of the fibrous material may be impregnated in such a way that there is a uniform or substantially uniform distribution of the precursor solution through the relevant portion or whole of the bulk of the fibrous material.

The hydrogel liquid is evaporated or cured after contacting (e.g. coating), in the manner described above. Where the hydrogel liquid is a polymer solution preferred curing is by means of irradiation including gamma, electron beam and light (visible and ultra-violet). When the hydrogel liquid is a monomer containing solution curing is preferably achieved via irradiation including gamma, electron beam and light (visible and ultra-violet). A preferred method utilises ultraviolet light.

Protective liners (for example polyolefin films, paper, polyester, which may be optionally siliconised) may be placed on either or both surfaces. However, in a preferred embodiment of this invention no liners are needed, because of the relatively low amount of hydrogel in the product. The lack of liners facilitates the subsequent fast and economic processing of the roll stock into product. The hydrogel-containing absorbent material may be rolled on itself without the need for protective interliners/ release layers.

The invention thereby makes available an absorbent article which can be made from hydrogel-containing roll stock comprising rolls 5 to 1000m in length. This roll-stock is preparable in a substantially continuous and non-batchwise process, with considerable economic advantages.

Furthermore, we have surprisingly found that there is a beneficial interaction between the hydrogel and the absorbent sheet member to which it is applied, in that a certain amount of water is apparently abstracted from the hydrogel by the absorbent sheet, causing a lowering of the observed water activity of the hydrogel. This provides evidence of an active water transport mechanism within the absorbent material of the present invention.

### Articles and Applications

The hydrogels present in the composites described herein may be adhesive or non-adhesive. When they are adhesive, they are typically tacky to the touch, and therefore lend themselves to applications where a certain degree of adhesion to mammalian (particularly human) skin is required. When the hydrogel composites described herein are non-adhesive, they typically have no or negligible tackiness to the touch.

The absorbent materials and articles according to the present invention may preferably be capable of being removed from the skin without undue pain, discomfort or irritation, and without leaving a substantial mark or residue on the skin.

The materials may thus suitably be used in a range of skin contact or covering articles and applications where the composite is brought into contact either with skin or with an intermediary member which interfaces between the material and the skin. The material may be unsupported or may be supported on a part of a larger article for some specific use, e.g. a backing structure.

Articles and applications include patches, tapes, bandages, devices and dressings of general utility or for specific uses, including without limitation biomedical, skin care, personal and body care, palliative and veterinary uses such as, for example, skin electrodes for diagnostic (e.g. ECG), stimulation (e.g. TENS), therapeutic (e.g. defibrillation) or electrosurgical (e.g. electrocauterisation) use; dressings and reservoirs for assisting wound and burn healing, wound and burn management, skin cooling, skin moisturizing, skin warming, aroma release or delivery, decongestant release or delivery, pharmaceutical and drug release or delivery, perfume release or delivery, fragrance release or delivery, scent release or delivery, and other skin contacting devices such as absorbent pads or patches for absorbing body fluids (e.g. lactation pads for nursing mothers), cosmetic device adhesives, hairpiece adhesives and clothing adhesives; and adhesive flanges and tabs for fecal collection receptacles, ostomy devices and other incontinence devices.

The articles incorporating the hydrogel-containing material according to the present invention may have any convenient shape or configuration. Particularly but not exclusively, the articles may be provided in any conventional shape or configuration for the category of articles concerned, or any approximation thereto. For example, articles in substantially sheet form may be square, rectangular, triangular, polygonal, circular, oval, ellipsoidal, irregular, any of the above with indentations, any of the above with projections, or an approximation to any of the above.

The articles incorporating the hydrogel-containing material according to the present invention may incorporate the said material as an island surrounded by other portions of that or those face(s) of the article of which the hydrogel containing material forms part, or the said composite may extend to one or more edge of such face(s). Where the hydrogel-containing material is an island surrounded by other portions of that or those face(s) of the article of which the hydrogel-containing material forms part, the surrounding portions may be provided with other adhesive materials such as conventional pressure sensitive adhesives, such as, for example, acrylate ester adhesives, e.g. to provide skin adhesion.

Articles (such as those mentioned above) incorporating the hydrogel-containing material according to the present invention may suitably comprise a support member, typically in sheet or substantially sheet form, which is suitably flexible, conformable to the skin, with which the hydrogel composite according to the present invention is associated. The support member may be perforated or non-perforated. The support member may be unitary in construction or constructed as a composite of multiple parts, e.g. a plurality of layers. The construction of the parts other than the hydrogel-containing material of the present invention may suitably be generally conventional. For example, the support member of a wound dressing or the like may suitably comprise a flexible water-permeable or water-impermeable backing layer or other structure, which may optionally incorporate other adhesives if desired, and/or an absorbent layer or other structure (e.g. a foam or other absorbent material). Such additional parts may suitably be formed in any suitable material conventionally used for such articles, including for example synthetic and natural materials, e.g. polymers such as polyurethane, polyolefins, hydrogels, or any combination thereof.

Articles comprising multiple parts - e.g. layers or sheets - may suitably include adhesives (e.g. acrylic adhesives) to bond the parts together, or the parts may be retained together in the article by partial melting together, by crimping, embossing or other mechanical retention method, or any combination thereof.

If desired, a part of an article or a complete article, such as a skin patch, wound or burn dressing, bandage or plaster can incorporate a system for generating an bioactive agent such as a pharmaceutically active agent or combination of agents (drug), an antimicrobial agent or combination of agents, an antiseptic agent or combination of agents, or an antibiotic agent or combination of agents. Such a system may, for example, be the Bioxzyme™ system mentioned above.

Parts of the articles which are adapted to contact a patient during use, and at least those portions of the article adjacent to the patient-contacting parts, may if desired be sterilised and may conveniently be stored in sterile packaging.

The hydrogel-containing materials according to the present invention, and articles incorporating them, may be provided for storage, transportation and before use with a release sheet overlying any adhesive portions. The release sheet may take any conventional form, e.g. a paper or plastics sheet which may suitably be coated with a non-stick material such as silicone or polytetrafluoroethylene.

However, as mentioned above, because of the low amounts of hydrogel present in the products according to the present invention, a release sheet may in many instances not be necessary, as the hydrogel - even when plasticised - is likely to be insufficiently tacky to pick up contamination or dirt.

If desired, other portions of the articles may also suitably be provided for storage, transportation and before use with a release sheet overlying any other portions. The release sheet may take any conventional form, e.g. a paper or plastics sheet which may suitably be coated with a non-stick material such as silicone or polytetrafluoroethylene. For example, a surface of an article such as skin dressing which in use is directed away from the wearer's skin may if desired be provided with a surface or surface material that benefits from protection before use. In that case, for example, the said surface or surface material can be protected for storage and transportation before use by the release layer, which can then be removed and discarded after the article has been applied to the wearer's skin.

### Examples

The following non-limiting examples are provided as further illustration of the present invention, but without limitation.

### General Method

A precursor solution comprising 70 parts by weight of a 58% aqueous solution of the sodium salt of acrylamidomethylpropanesulphonic acid (NaAMPS, LZ2405 Lubrizol), 30 parts glycerol and 0.14 parts of a 1 to 10 (by weight) mixture of Daracure 1173 photoinitiator (Ciba Speciality Chemicals) and IRR280 cross-linker (PEG400 diacrylate, UCB Chemicals) was dispensed from a slot die 120mm wide at a coat weight of 100g/m² onto a moving web of one of four non-woven fabrics from Lantor, in each case the fabric supported on a web of siliconised paper (Cotek) moving at 7m/s and cured with a NUVA Solo 30 medium pressure mercury arc lamp (GEW).

The four non-woven fabrics used were as follows, using the Lantor reference numbers:

### Example 1 - 50.03.18

### Example 2 - 47.02.09

### Example 3 - 71.01.06

### Example 4 - 46.09.03

### Example 5

The same method as for Examples 1 to 4 was used, except that the non-woven fabric from Lantor, reference 50.03.18, was used and the precursor solution was dispensed at a coat weight of 150g/m².

### Example 6

The same method as for Example 5 was used, except that the precursor solution was dispensed at a coat weight of 200g/m².

### Example 7

A precursor solution comprising 52 parts by weight of a 58% aqueous solution of the sodium salt of acrylamidomethyl-propanesulphonic acid (NaAMPS, LZ2405 Lubrizol), 48 parts water and 0.14 parts of a 1 to 10 (by weight) mixture of Daracure 1173 photoinitiator (Ciba Speciality Chemicals) and IRR280 cross-linker (PEG400 diacrylate, UCB Chemicals) was dispensed from a slot die 120mm wide at a coat weight of 100g/m² onto a moving web of a non-woven fabric from Freudenberg, reference M1561, supported on a moving web of siliconised paper (Cotek) moving at 7m/s and cured with a NUVA Solo 30 medium pressure mercury arc lamp (GEW).

### Example 8

A precursor solution comprising 70 parts by weight of a 58% aqueous solution of the sodium salt of acrylamidomethylpropanesulphonic acid (NaAMPS, LZ2405 Lubrizol), 30 parts glycerol and 0.14 parts of a 1 to 10 (by weight) mixture of Daracure 1173 photoinitiator (Ciba Speciality Chemicals) and IRR280 cross-linker (PEG400 diacrylate, UCB Chemicals) was dispensed from a slot die 120mm wide at a coat weight of 100g/m² onto a moving web of a non-woven fabric from Freudenberg, reference M1561 supported on a web of siliconised paper (Cotek) moving at 7m/s and cured with a NUVA Solo 30 medium pressure mercury arc lamp (GEW).

### Example 9

The same method as for Example 8 was used, except that the precursor solution was dispensed at a coat weight of 300g/m².

### Example 10

A precursor solution comprising 52 parts by weight of a 58% aqueous solution of the sodium salt of acrylamidomethylpropanesulphonic acid (NaAMPS, LZ2405 Lubrizol), 48 parts water and 0.14 parts of a 1 to 10 (by weight) mixture of Daracure 1173 photoinitiator (Ciba Speciality Chemicals) and IRR280 cross-linker (PEG400 diacrylate, UCB Chemicals) was dispensed from a slot die 120mm wide at a coat weight of 150g/m² onto a moving web of a non-woven fabric from Lantor, reference 50.03.18 supported on a moving web of siliconised paper (Cotek) moving at 7m/s and cured with a NUVA Solo 30 medium pressure mercury arc lamp (GEW).

### Example 11

A precursor solution comprising 70 parts by weight of a 58% aqueous solution of the sodium salt of acrylamidomethylpropanesulphonic acid (NaAMPS, LZ2405 Lubrizol), 30 parts glycerol and 0.14 parts of a 1 to 10 (by weight) mixture of Daracure 1173 photoinitiator (Ciba Speciality Chemicals) and IRR280 cross-linker (PEG400 diacrylate, UCB Chemicals) was dispensed from a slot die 120mm wide at a coat weight of 300g/m² onto a moving web of siliconised paper (Cotek) and a non-woven fabric from Freudenberg, reference M1561, was laid on top of the precursor solution, moving at 7m/s and cured with a NUVA Solo 30 medium pressure mercury arc lamp (GEW).

### Results and Discussion

All the materials produced in the Examples above had rapid uptake of saline fluid, less than 10 seconds for a 1 ml drop. The surfaces of the materials made in the above Examples were "dry" to the touch in the sense that the materials could be rolled up with front and back surfaces in direct contact with each other and unrolled without the need for protective interliners.

On exposure to saline solution the hydrogel treated surfaces of the above exemplified materials became moist and lubricious to the touch but not "wet".

Water activity measurements (Aqualab T3) performed on the materials made in the above Examples indicate that a partial dehydration, compared to hydrogels made in the absence of the absorbent layer occurs. In Example 1, for example, the hydrogel made directly from the precursor solution without the absorbent layer had a water activity of 0.68 compared to 0.57 when made with the absorbent layer.

The above broadly describes the present invention, without limitation. Variations and modifications as will be readily apparent to those of ordinary skill in this art are intended to be covered by this application and all subsequent patents.

## Claims

1. An absorbent material comprising a flexible, skin-conformable, moisture-absorbent sheet member, optionally a net member in sheet form overlying and associated with the absorbent sheet member on at least one face thereof, and a hydrogel disposed on at least one of the net member, when present, and the absorbent sheet member in an amount of less than about 500 g of hydrogel per square metre per face, wherein the aqueous saline absorbency rate of the absorbent material through the face on which the hydrogel is disposed is less than about 300 seconds.

2. An absorbent material according to claim 1, wherein the net member is absent.

3. An absorbent material according to claim 1, wherein the net member is present.

4. An absorbent material according to claim 3, wherein the net member is of a substantially more open foraminous structure than the absorbent sheet member.

5. An absorbent material according to claim 3 or claim 4, wherein the net member has perforation sizes of between about 0.01 and about 25 mm².

6. An absorbent material according to claim 5, wherein the net member has perforation sizes of between about 0.05 and about 10 mm².

7. An absorbent material according to claim 6, wherein the net member has perforations sizes of between about 0.1 and about 1 mm².

8. An absorbent material according to any one of claims 3 to 7, wherein the net member comprises one or more of polyolefins, polyamides, ethylene/vinyl acetate copolymers and combinations and blends thereof.

9. An absorbent material according to claim 8, wherein the net member comprises high density polyethylene.

10. An absorbent material according to any one of the preceding claims, wherein the aqueous saline absorbency rate is less than about 200 seconds.

11. An absorbent material according to any one of the preceding claims wherein the aqueous saline absorbency rate is less than about 100 seconds.

12. An absorbent material according to any one of the preceding claims, wherein the absorbent sheet member is a fibrous absorbent sheet member.

13. An absorbent material according to claim 12, wherein the fibrous absorbent sheet member is knitted, woven or non-woven.

14. An absorbent material according to any one of claims 1 to 11, wherein the absorbent sheet member is a non-fibrous absorbent sheet member.

15. An absorbent material according to claim 14, wherein the non-fibrous absorbent sheet member is a foam, membrane or film.

16. An absorbent material according to any one of the preceding claims, wherein the hydrogel does not occlude at least a substantial proportion of surface moisture entry pores or other openings of the absorbent sheet member.

17. An absorbent material according to any one of claims 3 to 16, wherein the hydrogel does not occlude at least a substantial proportion of through-perforations of the net member.

18. An absorbent article comprising the absorbent material according to any one of the preceding claims.

19. An absorbent article according to claim 18 which is a patch, tape, bandage, device or dressing.

20. An absorbent article according to claim 19, wherein a protective release layer for the hydrogel is absent.

21. An absorbent article according to claim 19, wherein the hydrogel is protected by a protective release layer.

22. An absorbent article according to any one of claims 19 to 21, for self-application.

23. An absorbent article according to any one of claims 18 to 22, comprising a system for generating a bioactive agent.

24. An absorbent article according to claim 23, wherein the bioactive agent is a pharmaceutically active agent or combination of agents, an antimicrobial agent or combination of agents, an antiseptic agent or combination of agents, or an antibiotic agent or combination of agents.

25. An absorbent article according to any one of claims 18 to 24, sterilised and packaged in sterile packaging.

26. A method of manufacturing an absorbent material or article according to any one of the preceding claims, comprising applying an appropriate amount of the hydrogel to at least one face of an appropriate absorbent sheet material or net member or both.

27. A method according to claim 26, wherein the application of the hydrogel is carried out before a manufacturing step comprising associating the absorbent and net members together.

28. A method according to claim 26, wherein the application of the hydrogel is carried out during a manufacturing step comprising associating the absorbent and net members together.

29. A method according to claim 26, wherein the application of the hydrogel is carried out after a manufacturing step comprising associating the absorbent and net members together.

30. A method according to any one of claims 26 to 29, wherein the application of the hydrogel is by coating, impregnating or a combination thereof.

31. A method according to any one of claims 26 to 30, wherein a precursor liquid (pregel) is applied to the absorbent sheet material or net member or both, allowed to accumulate on the absorbent sheet material or net member or both, and/or be absorbed into it/them, and subsequently treated so that the hydrogel is formed and/or deposited from the precursor liquid.
